# EUROPEAN PATENT APPLICATION

(11) **EP 1 439 590 A2**
(43) Date of publication of application: **21.07.2004**
(21) Application number: 03025516.0
(22) Date of filing: 07.11.2003
(51) Int. Cl.: H01L 51/30, H01B 1/12, H01M 4/60, G03G 5/07, C07C 333/04, C08G 61/12, C09K 19/38

(54) **Mono-, oligo- and poly-bis(thienyl) arylenes and their use as charge transport materials**

(30) Priority: 04.12.2002 EP 02027102
(71) Applicant: MERCK PATENT GmbH, 64293 Darmstadt (DE)
(72) Inventor: Heeney, Martin, Southampton SO14 6TQ (GB); Giles, Mark, Southampton SO15 2LE (GB); Thierney, Steven, Southampton SO15 7QW (GB); McCulloch, Iain, Southampton SO53 4LG (GB); Bailey, Clare, Southampton Hamshire SO18 4PP (GB)

(57) **Abstract**

The invention relates to the use of mono-, oligo- and poly-bis(thienyl) arylenes as charge transport materials or semiconductors in electrooptical, electronic and electroluminescent devices, to charge transport and semiconductor materials, components and devices comprising mono-, oligo- and poly-bis(thienyl) arylenes, and to novel mono-, oligo- and poly-bis(thienyl) arylenes.

## Description

### Field of Invention

The invention relates to the use of mono-, oligo- and poly-bis(thienyl) arylenes as semiconductors or charge transport materials in electrooptical, electronic and electroluminescent devices. The invention further relates to semiconductor and charge transport materials, components and devices comprising mono-, oligo- and poly-bis(thienyl) arylenes. The invention relates to novel mono-, oligo- and poly-bis(thienyl) arylenes.

### Background and Prior Art

Organic materials have recently shown promise as the active layer in organic based thin film transistors and organic field effect transistors [see H. E. Katz et al., *Acc. Chem*. *Res*., 2001, **34,** 5, 359]. Such devices have potential applications in smart cards, security tags and the switching element in flat panel displays. Organic materials are envisaged to have substantial cost advantages over their silicon analogues if they can be deposited from solution, as this enables a fast, large-area fabrication route.

The performance of the device is principally based upon the charge carrier mobility of the semiconducting material and the current on/off ratio, so the ideal semiconductor should have a low conductivity in the off state, combined with a high charge carrier mobility (> 1 x 10⁻³ cm² V⁻¹ s⁻¹). In addition, it is important that the semiconducting material is relatively stable to oxidation, i.e., it has a high ionisation potential, as oxidation leads to reduced device performance.

A known compound which has been shown to be an effective p-type semiconductor for OFETs is pentacene [see S. F. Nelson et al., *Appl*. *Phys. Lett*., 1998, **72**, 1854]. When deposited as a thin film by vacuum deposition, it was shown to have carrier mobilities in excess of 1 cm² V⁻¹ s⁻¹ with very high current on/off ratios greater than 10⁶. However, vacuum deposition is an expensive processing technique that is unsuitable for the fabrication of large-area films.

Regioregular head-to-tail poly(3-hexylthiophene) has been reported with charge carrier mobility between 1 x 10⁻⁵ and 4.5 x 10⁻² cm² V⁻¹ s⁻¹, but with a rather low current on/off ratio between 10 and 10³ [see Z. Bao et al., *Appl*. *Pys. Lett*., 1996, **69**, 4108]. This low on/off current is due in part to the low ionisation potential of the polymer, which can lead to oxygen doping of the polymer under ambient conditions, and a subsequent high off current [see H. Sirringhaus et al., *Adv. Solid State Phys.,* 1999, **39**, 101].

A high regioregularity leads to improved packing and optimised microstructure, leading to improved charge carrier mobility [see H. Sirringhaus et al., *Science*, 1998, **280,** 1741-1744; H. Sirringhaus et al., *Nature,* 1999, **401**, 685-688; and H. Sirringhaus, et al., *Synthetic Metals*, 2000, **111-112**, 129-132]. In general, poly(3-alkylthiophenes) show improved solubility and are able to be solution processed to fabricate large area films. However, poly(3-alkylthiophenes) have relatively low ionisation potentials and are susceptible to doping in air.

It is an aim of the present invention to provide new materials for use as semiconductors or charge transport materials, which are easy to synthesize, have high charge mobility, good processibility and oxidative stability.

Another aim of the invention is to provide new semiconductor and charge transport components, and new and improved electrooptical, electronic and electroluminescent devices comprising these components, like field effect transistors (FET) as components of integrated circuitry or of thin film transistors (TFT), and organic light emitting diode (OLED) applications like electroluminescent displays or backlights of liquid crystal displays.

Other aims of the invention are immediately evident to those skilled in the art from the following description.

The inventors have found that these aims can be achieved by using mono-, oligo- and poly-bis(thienyl) arylenes as semiconductors and charge transport materials.

Poly-3,3"-dialkyl-2,2':5',2"-terthiophenes (**1**) prepared via ferric chloride oxidative coupling have been described in WO 94/02530 and by M. C. Gallazi et al., *Synthetic Metals* 2002, **128,** 91.

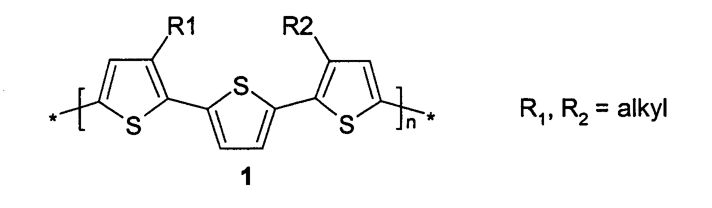

WO 94/02530, EP-A-0 945 723 and WO 99/31494 report the application of poly-3,3"-dialkyl-2,2':5',2"-terthiophenes (**1**) in their doped conductive form as electro-conductive layers in gas sensors. Furthermore, JP-A-63-002251 discloses conductive poly-3,3"-dialkyl-2,2':5',2"-terthiophenes (**1**) prepared via electrolytic polymerisation for use as polymeric cathodes in secondary batteries.

S. Holdcroft et al., *Macromolecules* 1999, **32**, 6889 discloses poly(bis(hexylthienyl)arylenes) (**2**) and (**3**) for use as electroluminescent materials.

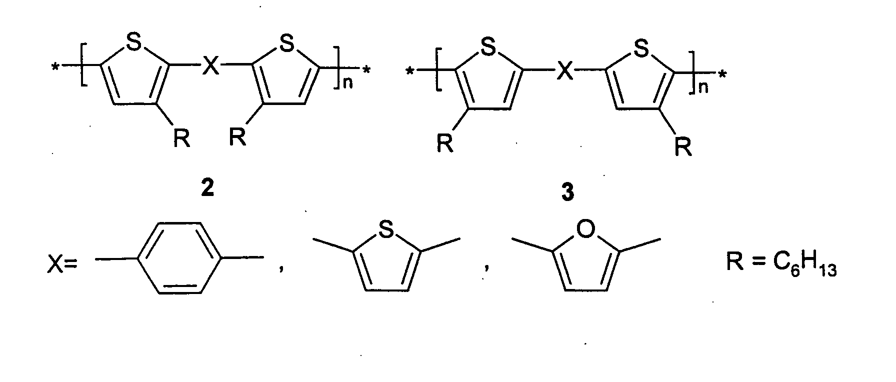

A. J. Heeger et al., *Macromolecules* 2001, **34**, 7241 discloses poly(bis(thienyl)arylenes) (**3**) for use as electroluminescent materials.

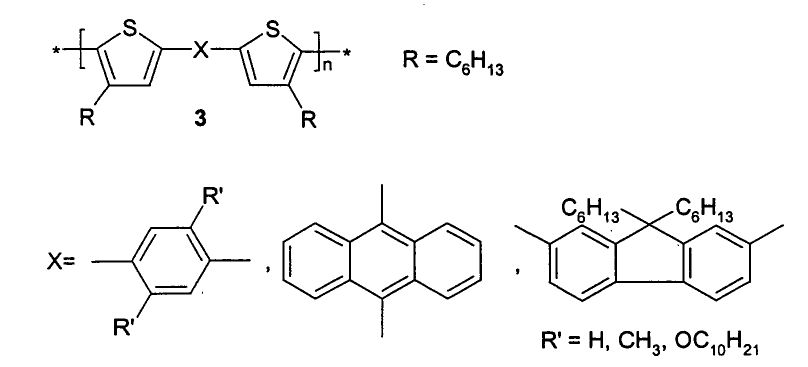

WO 01/78151 discloses poly(bis(thienyl)arylenes) (**4**) obtained by electrochemical polymerization for use in an organic light-emitting device.

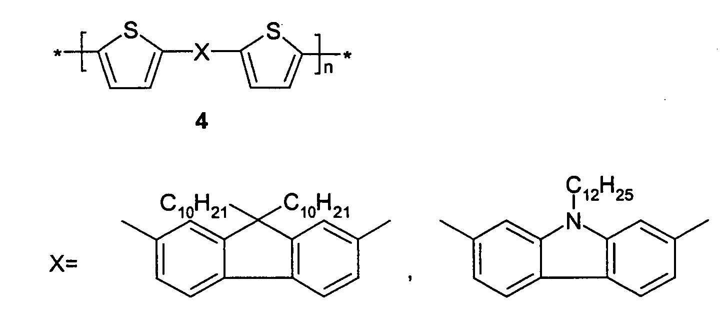

US 6,359,149 discloses conducting poly(bis(thienyl) arylenes) (**4**) and their use as electrode materials

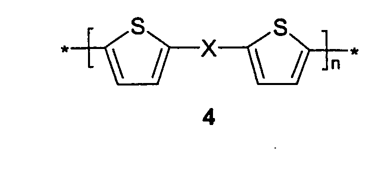

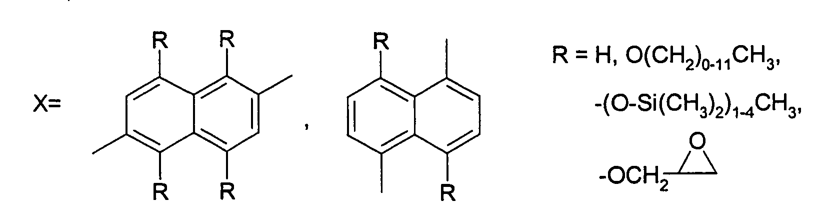

Reynolds et al., *Macromolecules* 1991, **24,** 678 and *Macromolecules* 1992, **25**, 849 disclose poly (bis(thienyl)arylenes) (**4a**) prepared via ferric chloride oxidative coupling and their doped conductive forms.

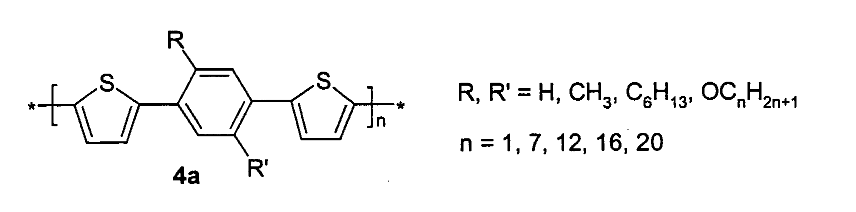

However, there have been no reports of the application of the above materials as semiconductors or charge transport materials.

Sirringhaus et al, *Appl. Phys. Lett.* 2000, **77**(3), 406 report the AB-type block-copolymer poly-9,9' dioctyl-fluorene-co-bithiophene 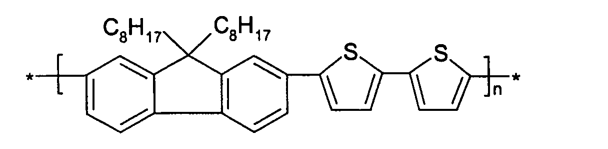 which has a thermotropic, nematic LC phase above 265 °C and can be oriented into a monodomain state to give improved field effect mobility, and its use in a TFT.

A further aspect of the invention relates to the synthesis of novel mono-, oligo- and poly-bis(thiophene) arylenes with improved properties, which are suitable as semiconductors or charge transport materials as well as for other uses.

A further aspect of the invention relates to reactive mesogens having a central core comprising a bis(thiophene) arylene unit, said core being linked, optionally via a spacer group, to one or two polymerisable groups. The reactive mesogens can induce or enhance liquid crystal phases or are liquid crystalline themselves. They can be oriented in their mesophase and the polymerisable group(s) can be polymerised or crosslinked in situ to form polymer films with a high degree of order, thus yielding improved semiconductor materials with high stability and high charge carrier mobility.

A further aspect of the invention relates to liquid crystal polymers like liquid crystal main chain or side chain polymers, in particular liquid crystal side chain polymers obtained from the reactive mesogens according to the present invention, which are then further processed, e.g., from solution as thin layers for use in semiconductor devices.

### Definition of Terms

The terms 'liquid crystalline or mesogenic material' or 'liquid. crystalline or mesogenic compound' means materials or compounds comprising one or more rod-shaped, lath-shaped or disk-shaped mesogenic groups, i.e., groups with the ability to induce liquid crystal phase behaviour. The compounds or materials comprising mesogenic groups do not necessarily have to exhibit a liquid crystal phase themselves. It is also possible that they show liquid crystal phase behaviour only in mixtures with other compounds, or when the mesogenic compounds or materials, or the mixtures thereof, are polymerised.

The term 'polymerisable' includes compounds or groups that are capable of participating in a polymerisation reaction, like radicalic or ionic chain polymerisation, polyaddition or polycondensation, and reactive compounds or reactive groups that are capable of being grafted for example by condensation or addition to a polymer backbone in a polymeranaloguous reaction.

The term 'film' includes self-supporting, i.e., free-standing, films that show more or less pronounced mechanical stability and flexibility, as well as coatings or layers on a supporting substrate or between two substrates.

### Summary of the Invention

The invention relates to the use of mono-, oligo- and polymers of formula I 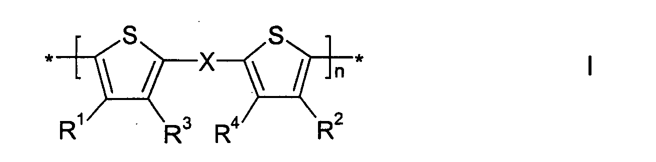 wherein
- X: is -CX¹=CX²-, -C≡C-, or arylene or heteroarylene that is optionally substituted with one or more groups R¹,
- X¹ and X²: are independently of each other H, F, Cl or CN,
- R¹⁻⁴: are independently of each other H, halogen, optionally substituted alkyl, cycloalkyl, aryl or heteroaryl, or P-Sp-,
- P: is a polymerisable or reactive group,
- Sp: is a spacer group or a single bond, and
- n: is an integer ≥ 1,
with the proviso that, if X is unsubstituted thiophene-2,5-diyl and R¹ and R² are H, then at least one of R³ and R⁴ is selected from alkyl or cycloalkyl that is mono-or polysubstituted by F, Cl, Br, I or CN, optionally substituted aryl or heteroaryl, and P-Sp-,
as semiconductors or charge transport materials.

The invention further relates to a semiconductor or charge transport material, component or device comprising at least one mono-, oligo- or polymer of formula I.

The invention further relates to the use of mono-, oligo- and polymers according to the present invention as semiconductors or charge transport materials in optical, electrooptical or electronic devices, like field effect transistors (FET) for example as components of integrated circuitry, of thin film transistors (TFT) for flat panel display applications, or of radio frequency identification (RFID) tags, or semiconducting components for organic light emitting diode (OLED) applications including both the charge transport and electroluminescent layers in electroluminescent displays or backlights of liquid crystal displays.

The invention further relates to novel mono-, oligo- or polymers of formula I, characterized in that they are selected from the following subformulae 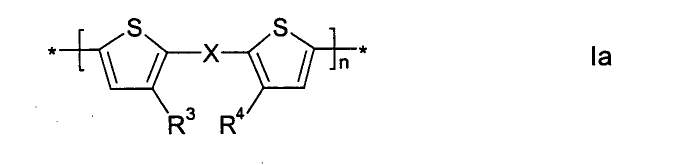 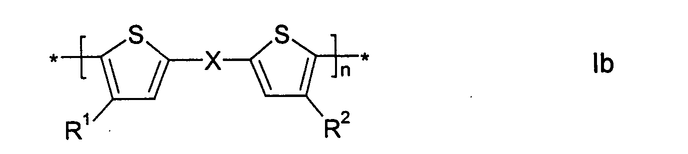 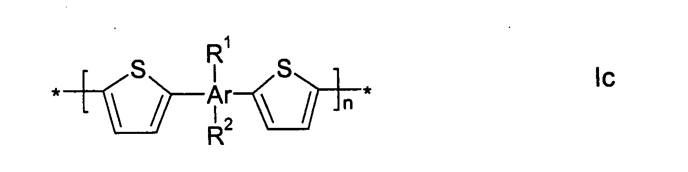 wherein X, n and R¹⁻⁴ are as defined in formula I, with R¹⁻⁴ being different from H, and Ar is arylene or heteroarylene, with the provisos that
a) if X or Ar is unsubstituted thiophene-2,5-diyl, then at least one of R¹⁻⁴ is alkyl or cycloalkyl that is mono- or polysubstituted by F, Cl, Br, I or CN, optionally substituted aryl or heteroaryl, or P-Sp-, and
b) X and Ar(R¹R²) are different from dithienothiophene, 1,4-phenylene, 2,5-dialkyl- or 2,5-dialkoxy-1,4-phenylene, furan-2,5-diyl, 1-alkyl-1 H-pyrrol-2,5-diyl, 9H-fluorene-2,7-diyl, 9,9-dialkyl-9H-fluorene-2,7-diyl, N-alkyl-9H-carbazole-2,7-diyl and anthracene-9,10-diyl, and
c) Ar(R¹R²) is different from 2,5-dialkyl- or 2,5-dialkoxy-1,4-phenylene, naphthalene-2,6-diyl, naphthalene-4,8-diyl that is substituted in 1-, 4-, 5- and/or 8-position with alkoxy, dimethylsiloxane or oxymethyloxirane groups, 9,9-dialkyl-9H-fluorene-2,7-diyl and N-alkyl-9H-carbazole-2,7-diyl.

The invention further relates to the use of the novel mono-, oligo- and polymers according to the present invention as electroluminescent materials, in photovoltaic or sensor devices, as electrode materials in batteries, as photoconductors and for electrophotographic applications like electrophotographic recording.

The invention further relates to an optical, electrooptical or electronic device, FET, integrated circuit (IC), TFT or OLED comprising a semiconducting or charge transport material, component or device according to the invention.

The invention further relates to a TFT or TFT array for flat panel displays, radio frequency identification (RFID) tag, electroluminescent display or backlight comprising a semiconducting or charge transport material, component or device or a FET, IC, TFT or OLED according to the invention.

The invention further relates to a security marking or device comprising a FET or an RFID tag according to the invention.

### Detailed Description of the Invention

In the polymers of formula I, copolymerisation of the substituted thiophene unit with other pi-conjugated species provides a method to move the position of the HOMO energy level. Making the HOMO level more negative and increasing the ionisation potential reduces the susceptibility to air oxidation and hence improves the stability. When used in a transistor, this reduces the OFF current of the transistor and therefore increases the ON/OFF ratio.

Regioregularity and morphology in thiophenes is of great importance for high mobility. An easy method to lock in regioregularity is to form polymers containing regioregular trimers made up of e.g. two alkylthiophenes and one other conjugated species. The centro symmetric nature of these units guarantees a regioregular synthesis.

Variation of the aromatic units X or Ar may also be used to enhance or induce liquid crystal behaviour in the polymers according to the present invention, which allows control of the morphology of the semiconductor in the transistor.

R¹⁻⁴ in formula I and Ia-c are preferably selected from H, halogen, straight chain, branched or cyclic alkyl with 1 to 20 C-atoms, which is unsubstituted, mono- or polysubstituted by F, Cl, Br, I or CN, and wherein one or more non-adjacent CH₂ groups are optionally replaced, in each case independently from one another, by -O-, -S-, - NH-, -NR⁰-, -SiR⁰R⁰⁰-, -CO-, -COO-, -OCO-, -OCO-O-, -SO₂-, -S-CO-, -CO-S-, -CH=CH- or -C≡C- in such a manner that O and/or S atoms are not linked directly to one another, optionally substituted aryl or heteroaryl and P-Sp-.
R⁰ and R⁰⁰ are independently of each other H or alkyl with 1 to 12 C-atoms.

If in the mono-, oligo- and polymers of formula I X is unsubstituted thiophene-2,5-diyl and R¹ and R² are H, then at least one, very preferably both of R³ and R⁴ are selected from straight chain, branched or cyclic alkyl with 1 to 20 C-atoms, which is mono- or polysubstituted by F, Cl, Br, I or CN, and wherein one or more non-adjacent CH₂ groups are optionally replaced, in each case independently from one another, by -O-, -S-, -NH-, -NR⁰-, -SiR⁰R⁰⁰-, - CO-, -COO-, -OCO-, -OCO-O-, -SO₂-, -S-CO-, -CO-S-, -CH=CH- or - C≡C- in such a manner that O and/or S atoms are not linked directly to one another, optionally substituted aryl or heteroaryl or P-Sp-.

The mono-, oligo- and polymers of formula I are preferably selected of formula I1 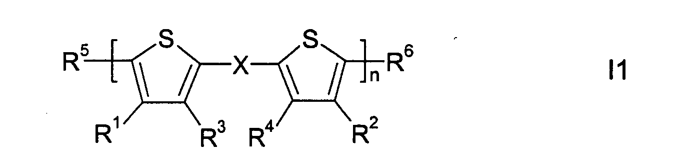 wherein R¹⁻⁴, X and n are as defined in formula I,
- R⁵ and R⁶: are independently of each other H, halogen, B(OR⁷)(OR⁸), SnR⁹R¹⁰R¹¹, straight chain, branched or cyclic alkyl with 1 to 20 C-atoms, which is unsubstituted, mono- or polysubstituted by F, Cl, Br, I or CN, and wherein one or more non-adjacent CH₂ groups are optionally replaced, in each case independently from one another, by -O-, -S-, -NH-, -NR⁰-, -SiR⁰R⁰⁰-, -CO-, -COO-, -OCO-, -OCO-O-, -SO₂-, -S-CO-, -CO-S-, -CH=CH- or -C≡C- in such a manner that O and/or S atoms are not linked directly to one another, optionally substituted aryl or heteroaryl and P-Sp-,
- R⁷ and R⁸: are independently of each other H or alkyl with 1 to 12 C-atoms, or OR⁷ and OR⁸ together with the boron atom form a cyclic group having 2 to 10 C atoms, and
- R⁹ to R¹¹: are independently of each other H or alkyl with 1 to 12 C-atoms.

The mono-, oligo- and polymers of formula Ia-c are preferably selected from the following formulae 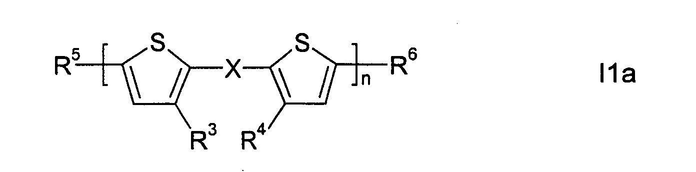 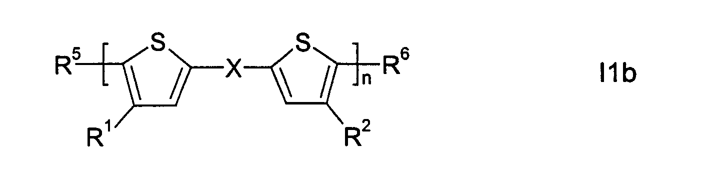 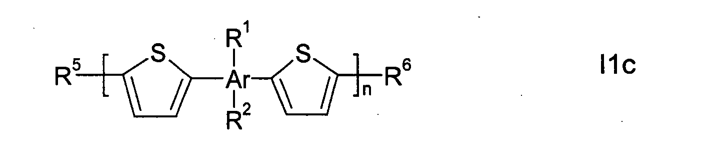 wherein R¹⁻⁶ , Ar, X and n have the meanings given above.

Particularly preferred are compounds of formula I1a-c wherein R¹ and R² or R³ and R⁴, respectively, have the same meaning.

Particularly preferred are mono-, oligo- and polymers of formulae I, I1, Ia-c and I1a-c wherein at least one of R¹⁻⁴ denotes an alkyl or fluoroalkyl group. The introduction of alkyl and fluoroalkyl groups improves the solubility and therefore the solution processibility of the inventive materials. Furthermore, the presence of fluoroalkyl groups also renders the inventive materials effective as *n*-type semiconductors.

Especially preferred are regioregular polymers of formula I and I1, in particular regioregular polymers of formulae la-c and I1a-c. The regioregularity in these polymers is preferably at least 90 %, in particular 95% or more, very preferably 98% or more, most preferably from 99 to 100%.

Regioregular polymers are advantageous as they show strong interchain pi-pi-stacking interactions and a high degree of crystallinity, making them effective charge transport materials with high carrier mobilities.

Further preferred are mono-, oligo- and polymers of formula I comprising at least one reactive group P that is capable of a polymerisation or crosslinking reaction.

Further preferred are mono-, oligo- and polymers of formula I that are mesogenic or liquid crystalline, in particular polymers forming calamitic phases, and reactive mesogens of formula I comprising one or more groups P-Sp-, forming calamitic phases.

Further preferred are mono-, oligo- and polymers of the formulae shown above and below wherein
- n is an integer from 1 to 5000,
- n is an integer from 2 to 5000, in particular from 20 to 1000,
- n is an integer from 2 to 5,
- n is an integer from 1 to 15 and one or both of R⁵ and R⁶ denote P-Sp-,
- at least one of R¹⁻⁶ denotes P-Sp-,
- n is an integer from 2 to 5000 and R⁵ and R⁶ are different from P-Sp-,
- the molecular weight is from 5000 to 100000,
- R¹⁻⁴ are selected from C₁-C₂₀-alkyl that is optionally substituted with one or more fluorine atoms, C₁-C₂₀-alkenyl, C₁-C₂₀-alkynyl, C₁-C₂₀-alkoxy, C₁-C₂₀-thioalkyl, C₁-C₂₀-silyl, C₁-C₂₀-ester, C₁-C₂₀-amino, C₁-C₂₀-fluoroalkyl, (CH₂CH₂O)ₘ with m being an integer from 1 to 6, and optionally substituted aryl or heteroaryl, very preferably C₁-C₂₀-alkyl or C₁-C₂₀-fluoroalkyl,
- X is -CX¹=CX²- or -C≡C-, with X¹ and X² preferably not being at the same time F,
- X in formula la, Ib, I1a and I1b is different from 1,4-phenylene, 2,5-dialkyl- or 2,5-dialkoxy-1,4-phenylene, furan-2,5-diyl, 1-alkyl-1 H-pyrrol-2,5-diyl, 9,9-dialkyl-9H-fluorene-2,7-diyl, N-alkyl-9H-carbazole-2,7-diyl and anthracene-9,10-diyl,
- n > 1.

X and Ar(R¹R²) are preferably mono-, bi- or tricyclic arylene or heteroarylene with up to 25 C atoms, wherein the rings can be fused, and in which the heteroaromatic groups contain at least one hetero ring atom, preferably selected from N, O and S. The arylene and heteroarylene groups are optionally substituted with one or more of F, Cl, Br, I, CN, and straight chain, branched or cyclic alkyl having 1 to 20 C atoms, which is unsubstituted, mono- or poly-substituted by F, Cl, Br, I, -CN or -OH, and in which one or more non-adjacent CH₂ groups are optionally replaced, in each case independently from one another, by -O-, -S-, -NH-, -NR⁰-, -SiR⁰R⁰⁰-, -CO-, -COO-, OCO-, - OCO-O, -S-CO-, -CO-S-,-CH=CH- or -C≡C- in such a manner that O and/or S atoms are not linked directly to one another.

X is preferably selected from fluorinated phenyl, pyridine, pyrimidine, biphenyl, naphthalene, 2,2'-bithiophene, fluorinated thiophene, benzo[1,2-b:4,5-b'] dithiophene, anthracene-2,6-diyl, thiazole and oxazole, all of which are unsubstituted, mono- or polysubstituted with L, wherein L is F, Cl, Br, or an alkyl, alkoxy, alkylcarbonyl or alkoxycarbonyl group with 1 to 12 C atoms, wherein one or more H atoms are optionally replaced by F or Cl.

Most preferably X in formula I and I1 is selected from the following formulae and their mirror images 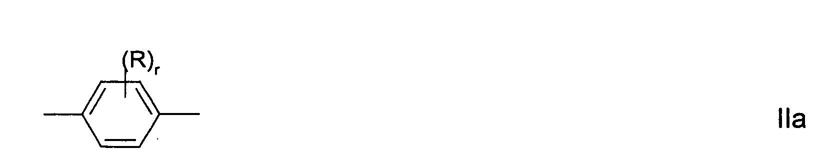 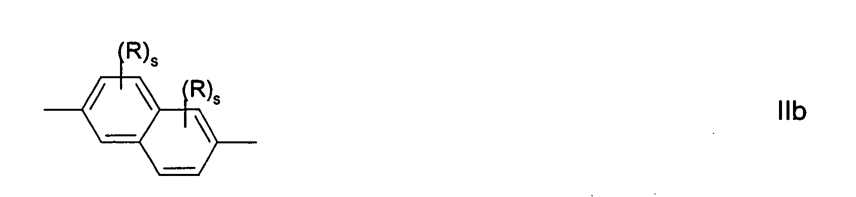 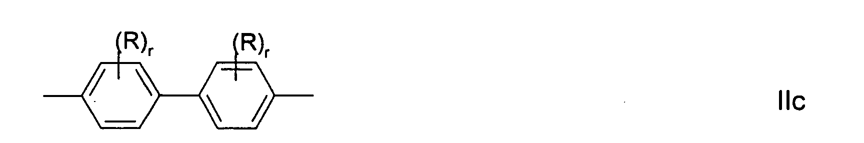 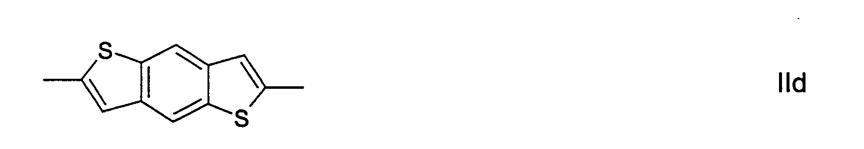 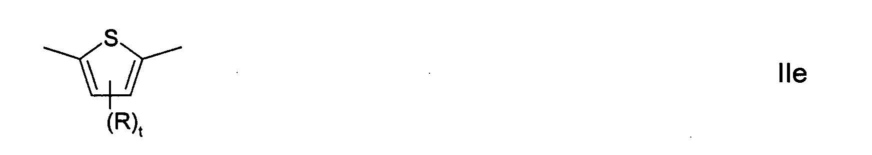 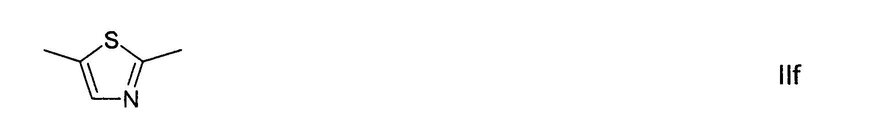 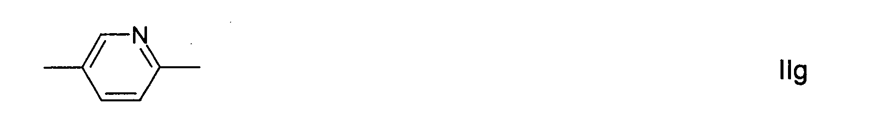 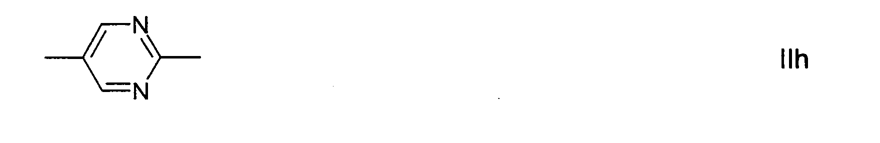 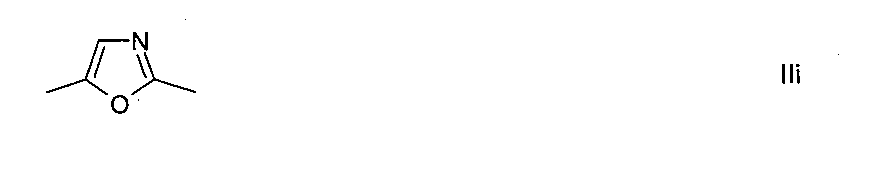 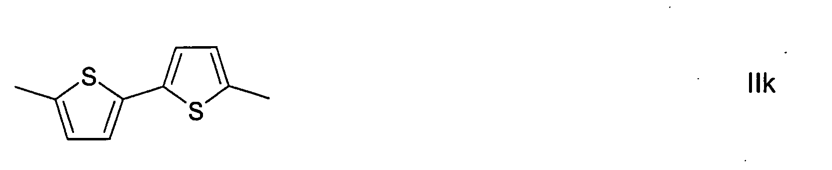 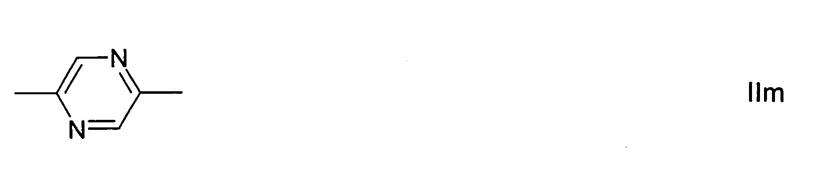 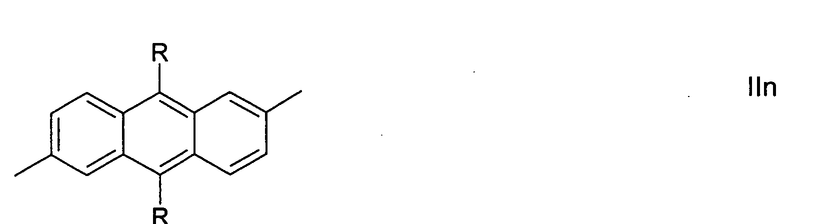 wherein R has one of the meanings of R¹ given above and preferably one of the meanings of L as defined above, r is 0, 1, 2, 3 or 4, s is 0, 1 , 2 or 3 and t is 1 or 2.

Especially preferred groups of formula IIa are 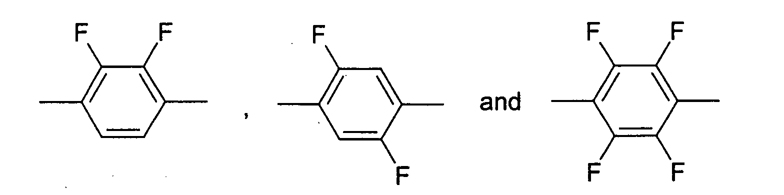 Ar(R¹R²) in formula Ic and I1c is most preferably selected from the following formulae and their mirror images 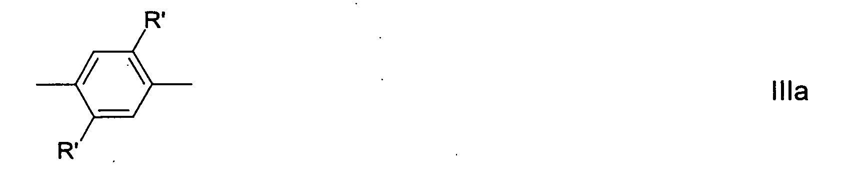 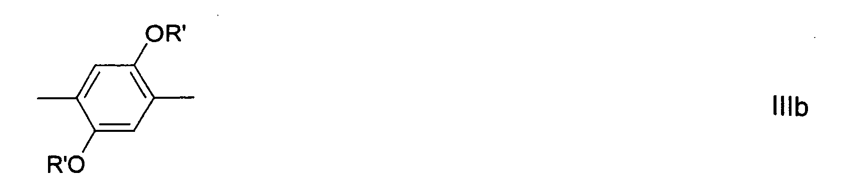 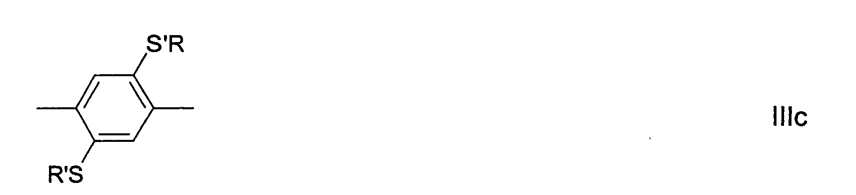 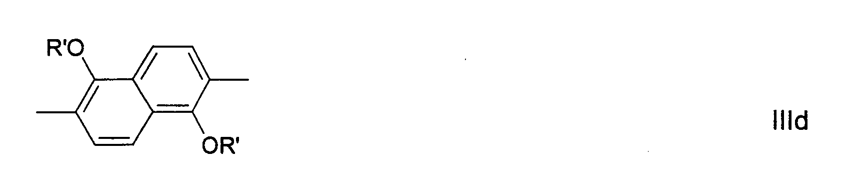 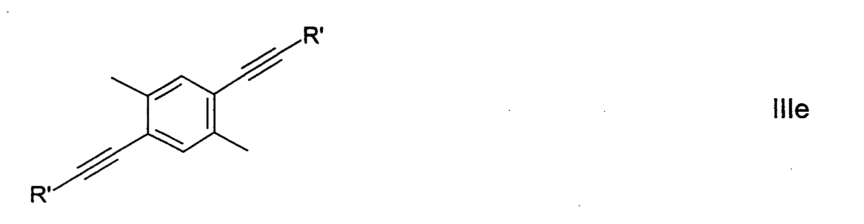 wherein R' has one of the meanings of R¹ in formula I and preferably has one of the meanings of L as defined above, very preferably alkyl, preferably straight chain alkyl, with 1 to 20 C atoms that is optionally fluorinated.

In the novel mono-, oligo- and polymers of formulae Ia-c X is most preferably selected from the above formulae Ila-h, in particular formulae Ilc, Ild, lie, IIf, IIi, IIj and III, wherein R is preferably F, Cl, alkyl or fluorinated alkyl or alkoxy with 1 to 15 C atoms.

In the novel mono-, oligo- and polymers of formulae Ia-c Ar is most preferably selected from the above formulae IIIc and IIIe, wherein R' is preferably straight chain alkyl with 1 to 15 C atoms that is optionally fluorinated, or from formulae IIIa, IIIb and IIId wherein R' is fluoroalkyl with 1 to 15 C atoms.

If one of R, R' and R¹⁻⁶ is aryl or heteroaryl, it is preferably a mono-, bi- or tricyclic aromatic or heteroaromatic group with up to 25 C atoms, wherein the rings can be fused, and in which the heteroaromatic group contains at least one hetero ring atom, preferably selected from N, O and S. It is optionally substituted with one or more of F, Cl, Br, I, CN, and straight chain, branched or cyclic alkyl having 1 to 20 C atoms, which is unsubstituted, mono- or poly-substituted by F, Cl, Br, I, -CN or -OH, and in which one or more non-adjacent CH₂ groups are optionally replaced, in each case independently from one another, by -O-, -S-, -NH-, -NR⁰-, -SiR⁰R⁰⁰-, - CO-, -COO-, OCO-, -OCO-O, -S-CO-, -CO-S-,-CH=CH- or -C≡C- in such a manner that O and/or S atoms are not linked directly to one another.

Especially preferred aryl and heteroaryl groups are phenyl, fluorinated phenyl, pyridine, pyrimidine, biphenyl, naphthalene, thiophene, fluorinated thiophene, benzo[1,2-b:4,5-b']dithiophene, thiazole and oxazole, all of which are unsubstituted, mono- or polysubstituted with L as defined above.

If in the formulae shown above and below one of R, R' and R¹⁻⁶ is an alkyl or alkoxy radical, i.e., where the terminal CH₂ group is replaced by -O-, this may be straight-chain or branched. It is preferably straight-chain, has 2 to 8 carbon atoms and accordingly is preferably ethyl, propyl, butyl, pentyl, hexyl, heptyl, octyl, ethoxy, propoxy, butoxy, pentoxy, hexyloxy, heptoxy, or octoxy, furthermore methyl, nonyl, decyl, undecyl, dodecyl, tridecyl, tetradecyl, pentadecyl, nonoxy, decoxy, undecoxy, dodecoxy, tridecoxy or tetradecoxy, for example.

Oxaalkyl, i.e., where one CH₂ group is replaced by -O-, is preferably straight-chain 2-oxapropyl (=methoxymethyl), 2- (=ethoxymethyl) or 3-oxabutyl (=2-methoxyethyl), 2-, 3-, or 4-oxapentyl, 2-, 3-, 4-, or 5-oxahexyl, 2-, 3-, 4-, 5-, or 6-oxaheptyl, 2-, 3-, 4-, 5-, 6- or 7-oxaoctyl, 2-, 3-, 4-, 5-, 6-, 7- or 8-oxanonyl or 2-, 3-, 4-, 5-, 6-,7-, 8- or 9-oxadecyl, for example.

Fluoroalkyl or fluorinated alkyl or alkoxy is preferably straight chain (O)CᵢF₂ᵢ₊₁, wherein i is an integer from 1 to 20, in particular from 1 to 15, very preferably (O)CF₃, (O)C₂F₅, (O)C₃F₇, (O)C₄F₉, (O)C₅F₁₁, (O)C₆F₁₃, (O)C₇F₁₅ or (O)C₈F₁₇, most preferably (O)C₆F₁₃.

Halogen is preferably F, Br or Cl.

Hetero atoms are preferably selected from N, O and S.

The polymerisable or reactive group P is preferably selected from CH₂=CW¹-COO-, 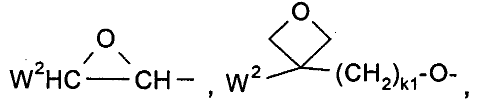 CH₂=CW²- (O)ₖ₁-, CH₃-CH=CH-O-, (CH₂=CH)₂CH-OCO-, (CH₂=CH)₂CH-O-, (CH₂=CH-CH₂)₂CH-OCO-, (CH₂=CH-CH₂)₂N-, HO-CW²W³-, HS-CW²W³-, HW²N-, HO-CW²W³-NH-, CH₂=CW¹-CO-NH-, CH₂=CH-(COO)ₖ₁-Phe-(O)ₖ₂-, Phe-CH=CH-, HOOC-, OCN-, and W⁴W⁵W⁶Si-, with W¹ being H, Cl, CN, phenyl or alkyl with 1 to 5 C-atoms, in particular H, Cl or CH₃, W² and W³ being independently of each other H or alkyl with 1 to 5 C-atoms, in particular methyl, ethyl or n-propyl, W⁴, W⁵ and W⁶ being independently of each other Cl, oxaalkyl or oxacarbonylalkyl with 1 to 5 C-atoms, Phe being 1,4-phenylene and k₁ and k₂ being independently of each other 0 or 1.

Especially preferred groups P are CH₂=CH-COO-, CH₂=C(CH₃)-COO-, CH₂=CH-, CH₂=CH-O-, (CH₂=CH)₂CH-OCO-, (CH₂=CH)₂CH-O-, and

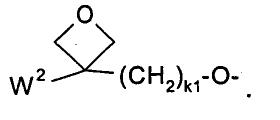

Very preferred are acrylate and oxetane groups. Oxetanes produce less shrinkage upon polymerisation (cross-linking), which results in less stress development within films, leading to higher retention of ordering and fewer defects. Oxetane cross-linking also requires cationic initiator, which unlike free radical initiator is inert to oxygen.

As for the spacer group Sp all groups can be used that are known for this purpose to the skilled in the art. The spacer group Sp is preferably of formula Sp'-X, such that P-Sp- is P-Sp'-X-, wherein
- Sp': is alkylene with up to 20 C atoms which may be unsubstituted, mono- or poly-substituted by F, Cl, Br, I or CN, it being also possible for one or more non-adjacent CH₂ groups to be replaced, in each case independently from one another, by -O-, - S-, -NH-, -NR⁰-, -SiR⁰R⁰⁰-, -CO-, -COO-, -OCO-, -OCO-O-, -S-CO-, -CO-S-, -CH=CH- or -C≡C- in such a manner that O and/or S atoms are not linked directly to one another,
- X: is -O-, -S-, -CO-, -COO-, -OCO-, -O-COO-, -CO-NR⁰-, -NR⁰-CO-, -OCH₂-, -CH₂O-, -SCH₂-, -CH₂S-, -CF₂O-, -OCF₂-, -CF₂S-, - SCF₂-, -CF₂CH₂-, -CH₂CF₂-, -CF₂CF₂-, -CH=N-, -N=CH-, -N=N-, -CH=CR⁰-, -CX¹=CX²-, -C≡C-, -CH=CH-COO-, -OCO-CH=CH- or a single bond, and
- R⁰, R⁰⁰, X¹ and X²: have one of the meanings given above.

X is preferably -O-, -S-, -OCH₂-, -CH₂O-, -SCH₂-, -CH₂S-, -CF₂O-, - OCF₂-, -CF₂S-, -SCF₂-, -CH₂CH₂-, -CF₂CH₂-, -CH₂CF₂-, -CF₂CF₂-, - CH=N-, -N=CH-, -N=N-, -CH=CR⁰-, -CX¹=CX²-, -C≡C- or a single bond, in particular -O-, -S-, -C≡C-, -CX¹=CX²- or a single bond, very preferably a group that is able to from a conjugated system, such as -C≡C- or -CX¹=CX²-, or a single bond.

Typical groups Sp' are, for example, -(CH₂)ₚ-, -(CH₂CH₂O)_{q}-CH₂CH₂-, - CH₂CH₂-S-CH₂CH₂- or -CH₂CH₂-NH-CH₂CH₂- or -(SiR⁰R⁰⁰-O)ₚ-, with p being an integer from 2 to 12, q being an integer from 1 to 3 and R⁰ and R⁰⁰ having the meanings given above.

Preferred groups Sp' are ethylene, propylene, butylene, pentylene, hexylene, heptylene, octylene, nonylene, decylene, undecylene, dodecylene, octadecylene, ethyleneoxyethylene, methyleneoxybutylene, ethylene-thioethylene, ethylene-N-methyl-iminoethylene, 1-methylalkylene, ethenylene, propenylene and butenylene for example.

Further preferred are compounds with one or two groups P-Sp- wherein Sp is a single bond.

In case of compounds with two groups P-Sp, each of the two polymerisable groups P and the two spacer groups Sp can be identical or different.

SCLCPs obtained from the inventive compounds or mixtures by polymerisation or copolymerisation have a backbone that is formed by the polymerisable group P.

The mono-, oligo- and polymers of the present invention can be synthesized according to or in analogy to known methods. Some preferred methods are described below.

Two general methods are possible for making the polymers of the present invention: polymerisation of a preformed trimer and direct polymerisation using two comonomers.

### Method 1: Polymerisation of the Trimer

Synthesis of the trimers can be carried out in a number of different ways. A number of reactive organometalic species of alkylthiophenes can be reacted with dihalo aromatic species under transition metal catalysis to give the trimer as shown in **Scheme 1**. This can also be reversed with a halo alkylthiophene being reacted with a diorganometalic species. 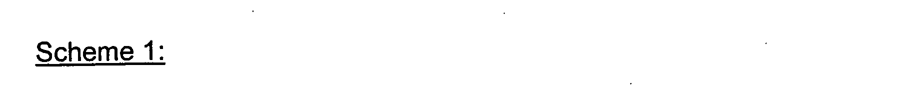 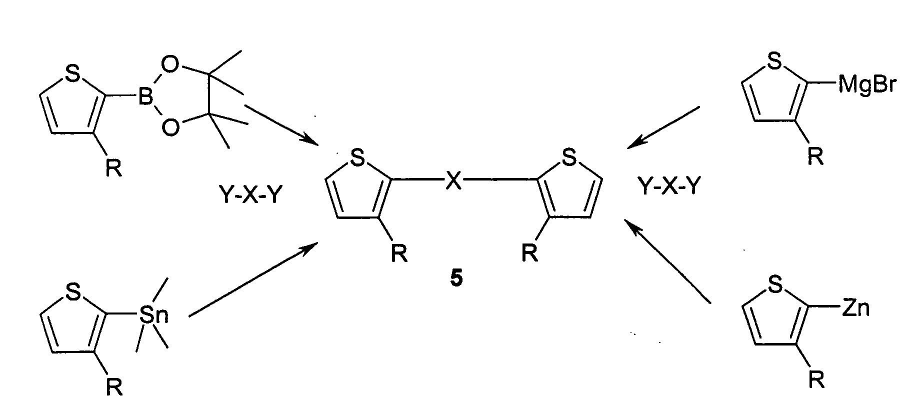 wherein X, Y and R have the meanings given above and Y is halogen, preferably Br or I.

Once formed the trimer (**5**) can be directly polymerised by oxidative polymerisation or can be further derivatised to the dihalo (**6**) species followed by polymerisation by one of several methodologies, for example the Yamomoto method [Yamamoto, T.; Morita, A;' Miyazaki, Y.; Maruyama, T.; Wakayama, H.; Zhou, Z.; Nakamura, Y.;. Kanbara, T.; Sasaki, S.; Kubota K. macromolecules 1992, 25, 1214] or McCullough method [Loewe, R. S.; Khersonsky, S. M.; McCullough, R. D. **1999**] as shown in **Scheme 2.**

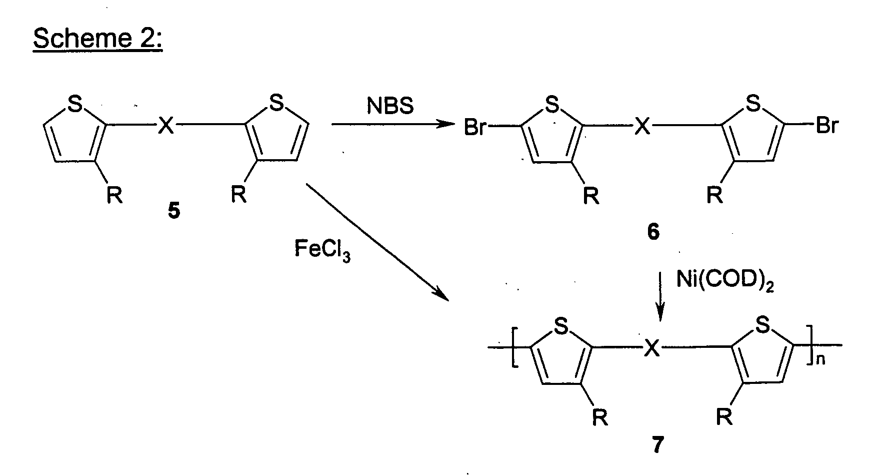

### Method 2: Cross coupling

An alternative route to these polymers is to carry out a cross coupling polymerisation as shown in **Scheme 3**. The dibromobithiophene (**8** or **10**) is reacted with either a bis stannyl or a bis boronic acid aromatic species in the presence of a transition metal catalyst to give the polymer (**9** or **7**).

Similar methologies to those shown above can be used to synthesise polymers of formula Ic.

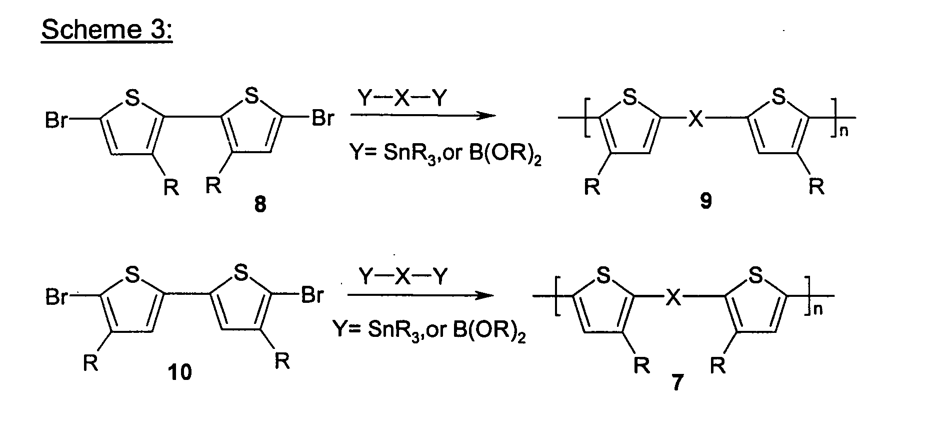

A further aspect of the invention relates to both the oxidised and reduced form of the compounds and materials according to this invention. Either loss or gain of electrons results in formation of a highly delocalised ionic form, which is of high conductivity. This can occur on exposure to common dopants. Suitable dopants and methods of doping are known to those skilled in the art, e.g., from EP 0 528 662, US 5,198,153 or WO 96/21659.

The doping process typically implies treatment of the semiconductor material with an oxidating or reducing agent in a redox reaction to form delocalised ionic centres in the material, with the corresponding counterions derived from the applied dopants. Suitable doping methods comprise for example exposure to a doping vapor in the atmospheric pressure or at a reduced pressure, electrochemical doping in a solution containing a dopant, bringing a dopant into contact with the semiconductor material to be thermally diffused, and ion-implantantion of the dopant into the semiconductor material.

When electrons are used as carriers, suitable dopants are for example halogens (e.g., I₂, Cl₂, Br₂, ICl, ICl₃, IBr and IF), Lewis acids (e.g., PF₅, AsF₅, SbF₅, BF₃, BCl₃, SbCl₅, BBr₃ and SO₃), protonic acids, organic acids, or amino acids (e.g., HF, HCl, HNO₃, H₂SO₄, HClO₄, FSO₃H and ClSO₃H), transition metal compounds (e.g., FeCl₃, FeOCl, Fe(ClO₄)₃, Fe(4-CH₃C₆H₄SO₃)₃, TiCl₄, ZrCl₄, HfCl₄, NbF₅, NbCl₅, TaCl₅, MoF₅, MoCl₅, WF₅, WCl₆, UF₆ and LnCl₃ (wherein Ln is a lanthanoid), anions (e.g., Cl⁻, Br⁻, I⁻, I₃⁻, HSO₄⁻, SO₄²⁻, NO₃⁻, ClO₄⁻, BF₄⁻, PF₆⁻, AsF₆⁻, SbF₆⁻, FeCl₄⁻, Fe(CN)₆³⁻, and anions of various sulfonic acids, such as aryl-SO₃⁻). When holes are used as carriers, examples of dopants are cations (e.g., H⁺, Li⁺, Na⁺, K⁺, Rb⁺ and Cs⁺), alkali metals (e.g., Li, Na, K, Rb, and Cs), alkaline-earth metals (e.g., Ca, Sr, and Ba), O₂, XeOF₄, (NO₂⁺) (SbF₆⁻), (NO₂⁺) (SbCl₆⁻), (NO₂⁺) (BF₄⁻), AgClO₄, H₂IrCl₆, La(NO₃)₃ · 6H₂O, FSO₂OOSO₂F, Eu, acetylcholine, R₄N⁺, (R is an alkyl group), R₄P⁺ (R is an alkyl group), R₆As⁺ (R is an alkyl group), and R₃S⁺ (R is an alkyl group).

The conducting form of the compounds and materials of the present invention can be used as an organic "metal" in applications, for example, but not limited to, charge injection layers and ITO planarising layers in organic light emitting diode applications, films for flat panel displays and touch screens, antistatic films, printed conductive substrates, patterns or tracts in electronic applications such as printed circuit boards and condensers.

A preferred embodiment of the present invention relates to mono-, oligo- and polymers of formula I and its preferred subformulae that are mesogenic or liquid crystalline, and very preferably comprise one or more polymerisable groups. Very preferred materials of this type are monomers and oligomers of formula I and its preferred subformulae wherein n is an integer from 1 to 15 and R⁵ and/or R⁶ denote P-Sp-.

These materials are particularly useful as semiconductors or charge transport materials, as they can be aligned into uniform highly ordered orientation in their liquid crystal phase by known techniques, thus exhibiting a higher degree of order that leads to particularly high charge carrier mobility. The highly ordered liquid crystal state can be fixed by in situ polymerisation or crosslinking via the groups P to yield polymer films with high charge carrier mobility and high thermal, mechanical and chemical stability.

For example, if a device is made from a polymerisable liquid crystal material by polymerisation in situ, the liquid crystal material preferably comprises one or more mono- or oligomers of formula I and its preferred subformulae wherein one or both of R⁵ and R⁶ denote P-Sp-. If a liquid crystal polymer is preapred first, for example by polymerisation in solution, and the isolated polymer is used to make the device, the polymer is preferably made from a liquid crystal material comprising one or more mono- or oligomers of formula I and its preferred subformulae wherein one of R⁵ and R⁶ denotes P-Sp-.

It is also possible to copolymerise the polymerisable mono-, oligo- and polymers according to the present invention with other polymerisable mesogenic or liquid crystal monomers that are known from prior art, in order to induce or enhance liquid crystal phase behaviour.

Thus, another aspect of the invention relates to a polymerisable liquid crystal material comprising one or more mono-, oligo- or polymers of the present invention as described above and below comprising at least one polymerisable group, and optionally comprising one or more further polymerisable compounds, wherein at least one of the polymerisable mono-, oligo- and polymers of the present invention and/or the further polymerisable compounds is mesogenic or liquid crystalline.

Particularly preferred are liquid crystal materials having a nematic and/or smectic phase. For FET applications smectic materials are especially preferred. For OLED applications nematic or smectic materials are especially preferred.

Another aspect of the present invention relates to an anisotropic polymer film with charge transport properties obtainable from a polymerisable liquid crystal material as defined above that is aligned in its liquid crystal phase into macroscopically uniform orientation and polymerised or crosslinked to fix the oriented state.

Preferably polymerisation is carried out as in-situ polymerisation of a coated layer of the material, preferably during fabrication of the electronic or optical device comprising the inventive semiconductor material. In case of liquid crystal materials, these are preferably aligned in their liquid crystal state into homeotropic orientation prior to polymerisation, where the conjugated pi-electron systems are orthogonal to the direction of charge transport. This ensures that the intermolecular distances are minimised and hence then energy required to transport charge between molecules is minimised. The molecules are then polymerised or crosslinked to fix the uniform orientation of the liquid crystal state. Alignment and curing are carried out in the liquid crystal phase or mesophase of the material. This technique is known in the art and is generally described for example in D.J. Broer, et al., Angew. Makromol. Chem. 183, (1990), 45-66

Alignment of the liquid crystal material can be achieved for example by treatment of the substrate onto which the material is coated, by shearing the material during or after coating, by application of a magnetic or electric field to the coated material, or by the addition of surface-active compounds to the liquid crystal material. Reviews of alignment techniques are given for example by I. Sage in "Thermotropic Liquid Crystals", edited by G. W. Gray, John Wiley & Sons, 1987, pages 75-77, and by T. Uchida and H. Seki in "Liquid Crystals - Applications and Uses Vol. 3", edited by B. Bahadur, World Scientific Publishing, Singapore 1992, pages 1-63. A review of alignment materials and techniques is given by J. Cognard, Mol. Cryst. Liq. Cryst. 78, Supplement 1 (1981), pages 1-77.

Polymerisation takes place by exposure to heat or actinic radiation. Actinic radiation means irradiation with light, like UV light, IR light or visible light, irradiation with X-rays or gamma rays or irradiation with high energy particles, such as ions or electrons. Preferably polymerisation is carried out by UV irradiation at a non-absorbing wavelength. As a source for actinic radiation for example a single UV lamp or a set of UV lamps can be used. When using a high lamp power the curing time can be reduced. Another possible source for actinic radiation is a laser, like e.g. a UV laser, an IR laser or a visible laser.

Polymerisation is preferably carried out in the presence of an initiator absorbing at the wavelength of the actinic radiation. For example, when polymerising by means of UV light, a photoinitiator can be used that decomposes under UV irradiation to produce free radicals or ions that start the polymerisation reaction. When curing polymerisable materials with acrylate or methacrylate groups, preferably a radical photoinitiator is used, when curing polymerisable materials with vinyl, epoxide and oxetane groups, preferably a cationic photoinitiator is used. It is also possible to use a polymerisation initiator that decomposes when heated to produce free radicals or ions that start the polymerisation. As a photoinitiator for radical polymerisation for example the commercially available Irgacure 651, Irgacure 184, Darocure 1173 or Darocure 4205 (all from Ciba Geigy AG) can be used, whereas in case of cationic photopolymerisation the commercially available UVI 6974 (Union Carbide) can be used.

The polymerisable material can additionally comprise one or more other suitable components such as, for example, catalysts, sensitizers, stabilizers, inhibitors, chain-transfer agents, co-reacting monomers, surface-active compounds, lubricating agents, wetting agents, dispersing agents, hydrophobing agents, adhesive agents, flow improvers, defoaming agents, deaerators, diluents, reactive diluents, auxiliaries, colourants, dyes or pigments.

Mono-, oligo- and polymers comprising one or more groups P-Sp- can also be copolymerised with polymerisable mesogenic compounds to induce or enhance liquid crystal phase behaviour. Polymerisable mesogenic compounds that are suitable as comonomers are known in prior art and disclosed for example in WO 93/22397; EP 0,261,712; DE 195,04,224; WO 95/22586 and WO 97/00600.

Another aspect of the invention relates to a liquid crystal side chain polymer (SCLCP) obtained from a polymerisable liquid crystal material as defined above by polymerisation or polymeranaloguous reaction. Particularly preferred are SCLCPs obtained from one or more monomers of formula I1 and its preferred subformulae wherein one or both of R⁵ and R⁶ are a polymerisable or reactive group, or from a polymerisable mixture comprising one or more of said monomers.

Another aspect of the invention relates to an SCLCP obtained from one or more monomers of formula I1 and its preferred subformulae wherein one or both of R⁵ and R⁶ are a polymerisable group, or from a polymerisable liquid crystal mixture as defined above, by copolymerisation or polymeranaloguous reaction together with one or more additional mesogenic or non-mesogenic comonomers.

Side chain liquid crystal polymers or copolymers (SCLCPs), in which the semiconducting component is located as a pendant group, separated from a flexible backbone by an aliphatic spacer group, offer the possibility to obtain a highly ordered lamellar like morphology. This structure consists of closely packed conjugated aromatic mesogens, in which very close (typically < 4 Å) pi-pi stacking can occur. This stacking allows intermolecular charge transport to occur more easily, leading to high charge carrier mobilities. SCLCPs are advantageous for specific applications as they can be readily synthesized before processing and then e.g. be processed from solution in an organic solvent. If SCLCPs are used in solutions, they can orient spontaneously when coated onto an appropriate surface and when at their mesophase temperature, which can result in large area, highly ordered domains.

SCLCPs can be prepared from the polymerisable compounds or mixtures according to the invention by the methods described above, or by conventional polymerisation techniques which are known to those skilled in the art, including for example radicalic, anionic or cationic chain polymerisation, polyaddition or polycondensation. Polymerisation can be carried out for example as polymerisation in solution, without the need of coating and prior alignment, or polymerisation in situ. It is also possible to form SCLCPs by grafting compounds according to the invention with a suitable reactive group, or mixtures thereof, to presynthesized isotropic or anisotropic polymer backbones in a polymeranaloguous reaction. For example, compounds with a terminal hydroxy group can be attached to polymer backbones with lateral carboxylic acid or ester groups, compounds with terminal isocyanate groups can be added to backbones with free hydroxy groups, compounds with terminal vinyl or vinyloxy groups can be added, e.g., to polysiloxane backbones with Si-H groups. It is also possible to form SCLCPs by copolymerisation or polymeranaloguous reaction from the inventive compounds together with conventional mesogenic or non mesogenic comonomers. Suitable comonomers are known to those skilled in the art. In principle it is possible to use all conventional comonomers known in the art that carry a reactive or polymerisable group capable of undergoing the desired polymer-forming reaction, like for example a polymerisable or reactive group P as defined above. Typical mesogenic comonomers are for example those mentioned in WO 93/22397, EP 0 261 712, DE 195 04 224, WO 95/22586, WO 97/00600 and GB 2 351 734. Typical non mesogenic comonomers are for example alkyl mono- or diacrylates or alkyl mono- or dimethacrylates with alkyl groups of 1 to 20 C atoms, like methyl acrylate or methyl methacrylate, trimethylpropane trimethacrylate or pentaerythritol tetraacrylate.

The mono-, oligo- and polymers of the present invention are useful as optical, electronic and semiconductor materials, in particular as charge transport materials in field effect transistors (FETs), e.g., as components of integrated circuitry, ID tags or TFT applications. Alternatively, they may be used in organic light emitting diodes (OLEDs) in electroluminescent display applications or as backlight of, e.g., liquid crystal displays, as photovoltaics or sensor materials, for electrophotographic recording, and for other semiconductor applications.

Especially the oligomers and polymers according to the invention show advantageous solubility properties which allow production processes using solutions of these compounds. Thus films, including layers and coatings, may be generated by low cost production techniques, e.g., spin coating. Suitable solvents or solvent mixtures comprise alkanes and/ or aromatics, especially their fluorinated derivatives.

The materials of the present invention are useful as optical, electronic and semiconductor materials, in particular as charge transport materials in field effect transistors (FETs), as photovoltaics or sensor materials, for electrophotographic recording, and for other semiconductor applications. Such FETs, where an organic semiconductive material is arranged as a film between a gate-dielectric and a drain and a source electrode, are generally known, e.g., from US 5,892,244, WO 00/79617, US 5,998,804, and from the references cited in the background and prior art chapter and listed below. Due to the advantages, like low cost production using the solubility properties of the compounds according to the invention and thus the processibility of large surfaces, preferred applications of these FETs are such as integrated circuitry, TFT-displays and security applications.

In security applications, field effect transistors and other devices with semiconductive materials, like transistors or diodes, may be used for ID tags or security markings to authenticate and prevent counterfeiting of documents of value like banknotes, credit cards or ID cards, national ID documents, licenses or any product with money value, like stamps, tickets, shares, cheques etc..

Alternatively, the mono-, oligo- and polymers according to the invention may be used in organic light emitting devices or diodes (OLEDs), e.g., in display applications or as backlight of e.g. liquid crystal displays. Common OLEDs are realized using multilayer structures. An emission layer is generally sandwiched between one or more electron-transport and/ or hole-transport layers. By applying an electric voltage electrons and holes as charge carriers move towards the emission layer where their recombination leads to the excitation and hence luminescence of the lumophor units contained in the emission layer. The inventive compounds, materials and films may be employed in one or more of the charge transport layers and/or in the emission layer, corresponding to their electrical and/ or optical properties. Furthermore their use within the emission layer is especially advantageous, if the compounds, materials and films according to the invention show electroluminescent properties themselves or comprise electroluminescent groups or compounds. The selection, characterization as well as the processing of suitable monomeric, oligomeric and polymeric compounds or materials for the use in OLEDs is generally known by a person skilled in the art, see, e.g., Meerholz, Synthetic Materials, 111-112, 2000, 31-34, Alcala, J. Appl. Phys., 88, 2000, 7124-7128 and the literature cited therein.

According to another use, the inventive compounds, materials or films, especially those which show photoluminescent properties, may be employed as materials of light sources, e.g., of display devices such as described in EP 0 889 350 A1 or by C. Weder et al., Science, 279, 1998, 835-837.

The invention is further explained by the following examples.

### Example 1

Polymer (**15**) was prepared as described below.

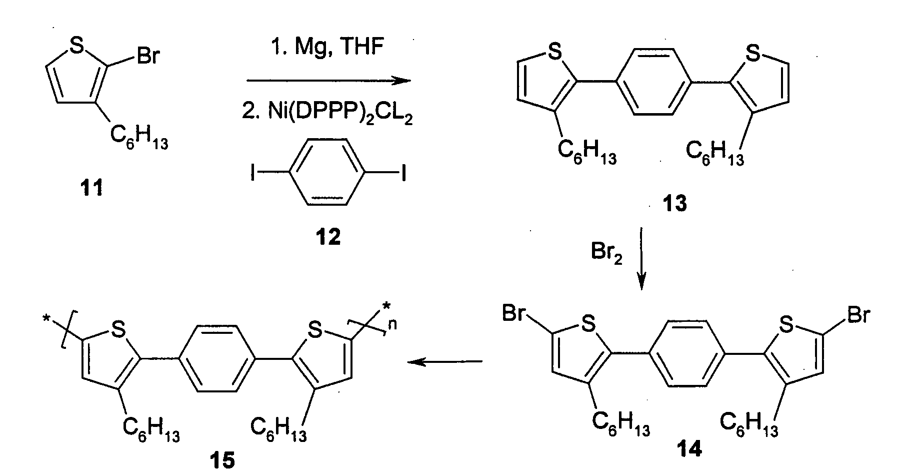

Diiodo benzene (**12**) was coupled to the Grignard reagent of 2-bromo, 3-hexylthiophene in the presence of a nickel catalyst to give the *ter*-aromatic species (**13**). This material was brominated to give the monomer(**14**). The monomer was polymerised by the Yamomoto methodology, using Nickel cyclooctadiene as catalyst to give the polymer (**15**). This material had a mobility of 1.8 x 10⁻⁵ cm²/VS and an ON/OFF ratio > 10⁴ and has a possible LC phase between 120 and 160 °C.

### Example 2

Polymer (**18**) was prepared as described below.

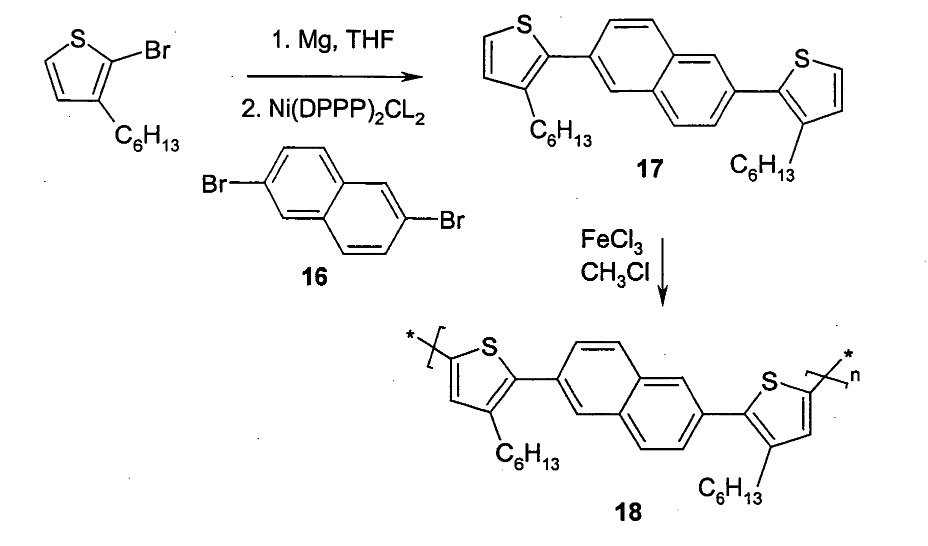

Dibromo naphthalene (**16**) was coupled to the Grignard reagent of 2-bromo, 3-hexylthiophene in the presence of a nickel catalyst to give the monomer (**17**). The monomer was polymerised by chemical oxidation using iron chloride to yield the polymer (**18**).

## Claims

1. Semiconductor or charge transport material, component or device comprising at least one mono-, oligo- or polymer of formula I 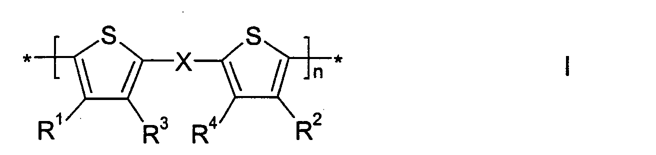 wherein
X is -CX¹=CX²-, -C≡C-, or optionally substituted arylene or heteroarylene,
X¹ and X² are independently of each other H, F, Cl or CN,
R¹⁻⁴ are independently of each other H, halogen, optionally substituted alkyl, cycloalkyl, aryl or heteroaryl, or P-Sp-,
P is a polymerisable or reactive group,
Sp is a spacer group or a single bond, and
n is an integer ≥ 1,
with the proviso that, if X is unsubstituted thiophene-2,5-diyl and R¹ and R² are H, then at least one of R³ and R⁴ is selected from alkyl or cycloalkyl that is mono-or polysubstituted by F, Cl, Br, I or CN, optionally substituted aryl or heteroaryl, and P-Sp-.

2. Semiconductor or charge transport material, component or device according to claim 1, **characterized in that** the mono-, oligo- or polymer is selected from the following formulae 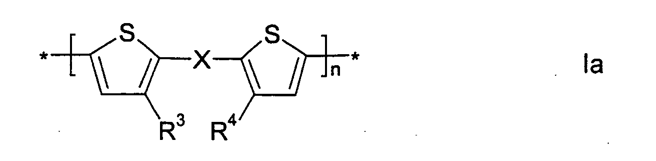 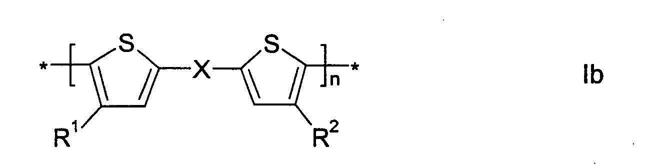 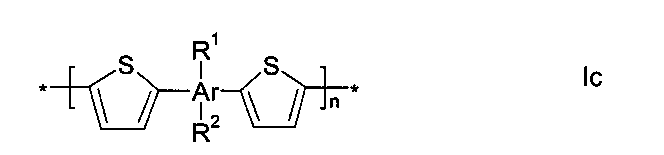 wherein X, n and R¹⁻⁴ are as defined in formula I, with R¹⁻⁴ being different from H, and Ar is arylene or heteroarylene.

3. Semiconductor or charge transport material, component or device according to at least one of claims 1 and 2, **characterized in that** the mono-, oligo- or polymer is selected of formula I1 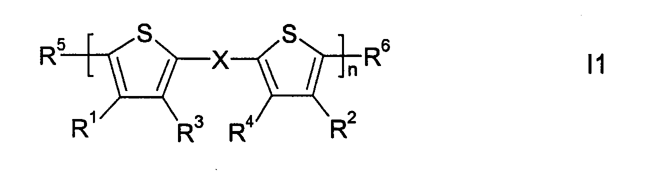 wherein R¹⁻⁴, X and n are as defined in formula I,
R⁵ and R⁶ are independently of each other H, halogen, B(OR⁷)(OR⁸), SnR⁹R¹⁰R¹¹, straight chain, branched or cyclic alkyl with 1 to 20 C-atoms, which is unsubstituted, mono- or polysubstituted by F, Cl, Br, I or CN, and wherein one or more non-adjacent CH₂ groups are optionally replaced, in each case independently from one another, by -O-, -S-, -NH-, -NR⁰-, -SiR⁰R⁰⁰-, -CO-, -COO-, -OCO-, -OCO-O-, -SO₂-, -S-CO-, -CO-S-, -CH=CH- or -C≡C- in such a manner that O and/or S atoms are not linked directly to one another, optionally substituted aryl or heteroaryl and P-Sp-,
R⁷ and R⁸ are independently of each other H or alkyl with 1 to 12 C-atoms, or OR⁷ and OR⁸ together with the boron atom form a cyclic group having 2 to 10 C atoms, and
R⁹ to R¹¹ are independently of each other H or alkyl with 1 to 12 C-atoms.

4. Semiconductor or charge transport material, component or device according to at least one of claims 1 to 3, **characterized in that** the mono-, oligo- or polymer is selected from the following formulae 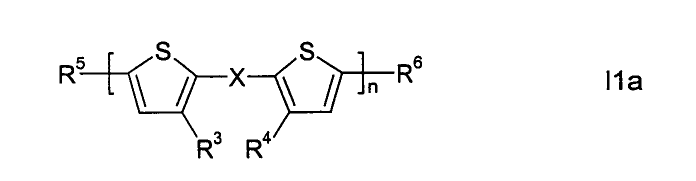 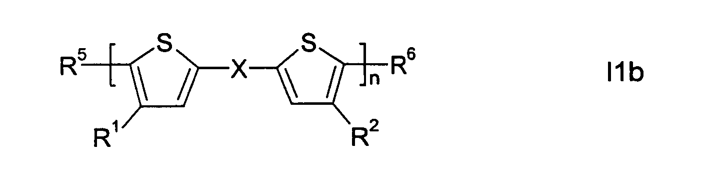 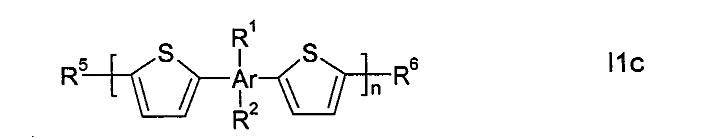 wherein R¹⁻⁶, X, Ar and n have the meanings given in formula la-c.

5. Semiconductor or charge transport material, component or device according to at least one of claims 1 to 4, **characterized**
**in that** the oligo- or polymer has a regioregularity of at least 95%.

6. Semiconductor or charge transport material, component or device according to at least one of claims 1 to 5, **characterized in that** n is an integer from 1 to 5000.

7. Semiconductor or charge transport material, component or device according to at least one of claims 1 to 6, **characterized in that** R¹⁻⁴ are selected from H, halogen, straight chain, branched or cyclic alkyl with 1 to 20 C-atoms, which is unsubstituted, mono- or polysubstituted by F, Cl, Br, I or CN, and wherein one or more non-adjacent CH₂ groups are optionally replaced, in each case independently from one another, by -O-, -S-, -NH-, -NR⁰-, -SiR⁰R⁰⁰-, -CO-, -COO-, - OCO-, -OCO-O-, -SO₂-, -S-CO-, -CO-S-, -CH=CH- or -C≡C- in such a manner that O and/or S atoms are not linked directly to one another, optionally substituted aryl or heteroaryl and P-Sp-, with R⁰ and R⁰⁰ being independently of each other H or alkyl with 1 to 12 C-atoms.

8. Semiconductor or charge transport material, component or device according to at least one of claims 1 to 7, **characterized in that** R¹⁻⁴ are selected from C₁-C₂₀-alkyl that is optionally substituted with one or more fluorine atoms, C₁-C₂₀-alkenyl, C₁-C₂₀-alkynyl, C₁-C₂₀-alkoxy, C₁-C₂₀-thioalkyl, C₁-C₂₀-silyl, C₁-C₂₀-ester, C₁-C₂₀-amino, C₁-C₂₀-fluoroalkyl, (CH₂CH₂O)ₘ with m being an integer from 1 to 6, and optionally substituted aryl or heteroaryl, very preferably C₁-C₂₀-alkyl or C₁-C₂ₒ-fluoroalkyl.

9. Semiconductor or charge transport material, component or device according to at least one of claims 1 to 8, **characterized in that** X and Ar(R¹R²) are mono-, bi- or tricyclic arylene or heteroarylene with up to 25 C atoms, wherein the rings can be fused, and in which the heteroaromatic groups contain at least one hetero ring atom, preferably selected from N, O and S. The arylene and heteroarylene groups are optionally substituted with one or more of F, Cl, Br, I, CN, and straight chain, branched or cyclic alkyl having 1 to 20 C atoms, which is unsubstituted, mono- or poly-substituted by F, Cl, Br, I, -CN or -OH, and in which one or more non-adjacent CH₂ groups are optionally replaced, in each case independently from one another, by -O-, - S-, -NH-, -NR⁰-, -SiR⁰R⁰⁰-, -CO-, -COO-, OCO-, -OCO-O, -S-CO-, -CO-S-,-CH=CH- or -C≡C- in such a manner that O and/or S atoms are not linked directly to one another.

10. Semiconductor or charge transport material, component or device according to at least one of claims 1 to 9, **characterized in that** X is selected from the following formulae and their mirror images 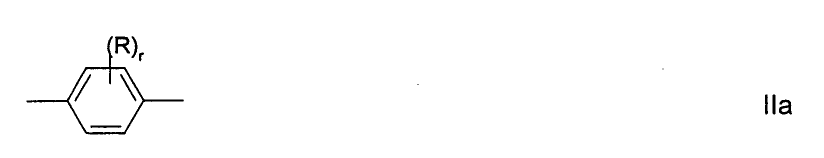 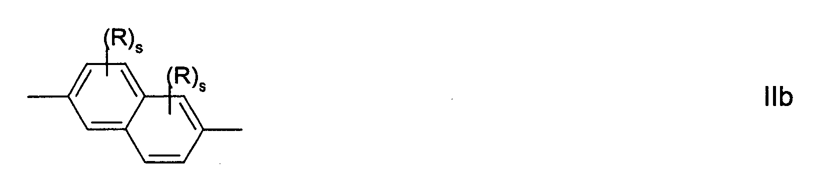 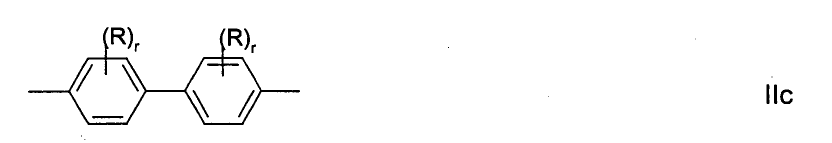 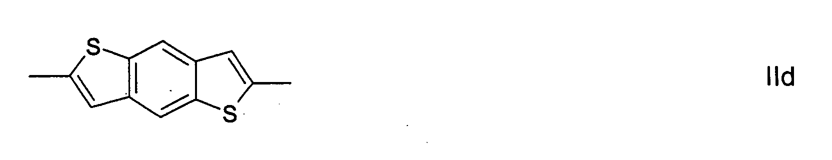 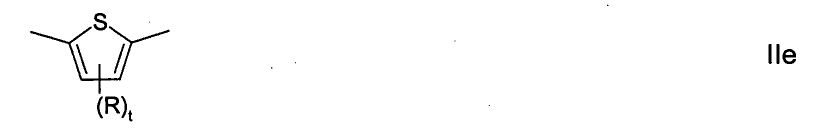 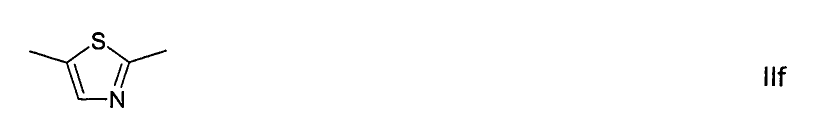 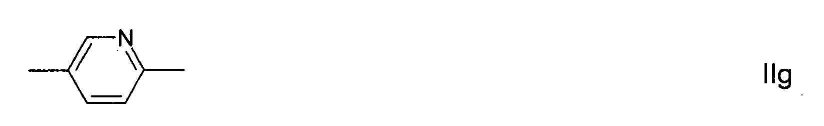 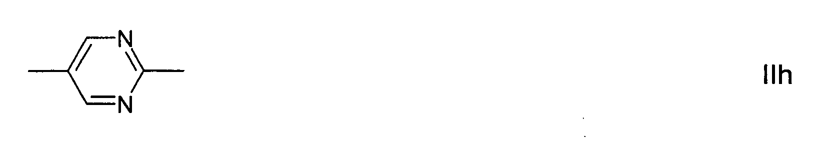 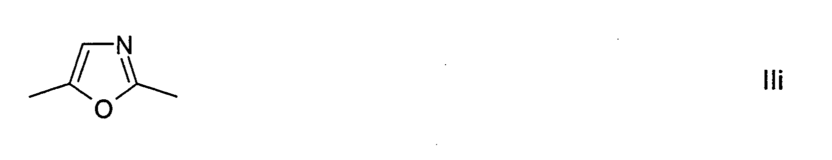 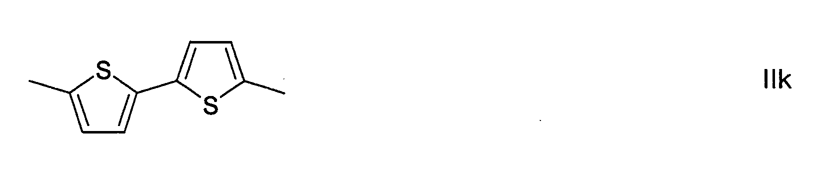 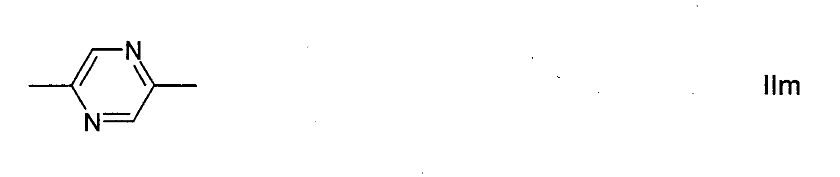 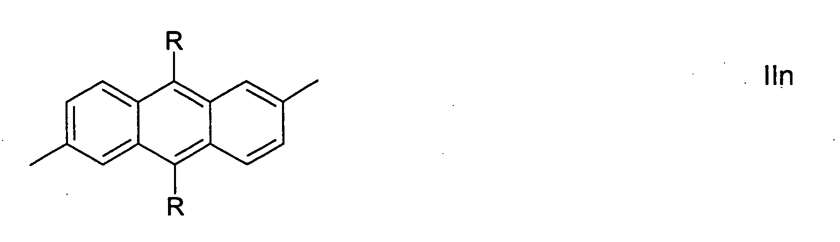 wherein R has one of the meanings of R¹ in formula I, r is 0, 1, 2, 3 or 4, s is 0, 1, 2 or 3 and t is 0, 1 or 2.

11. Semiconductor or charge transport material, component or device according to at least one of claims 1 to 10, **characterized**
**in that** Ar(R¹R²) is selected from the following formulae and their mirror images 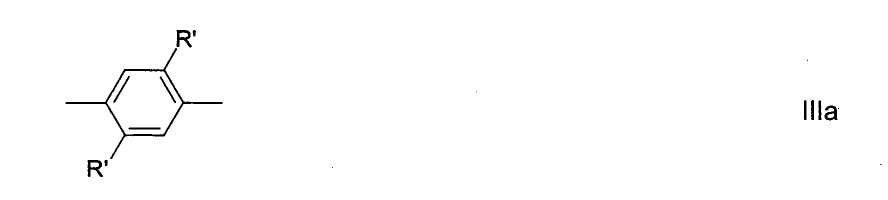 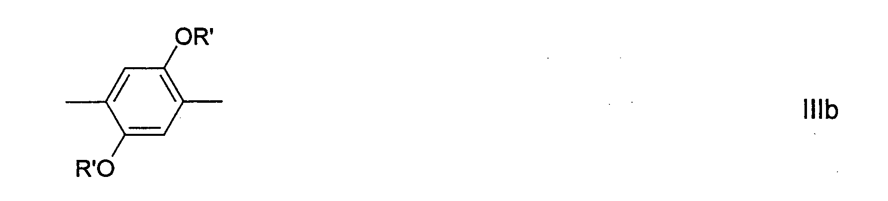 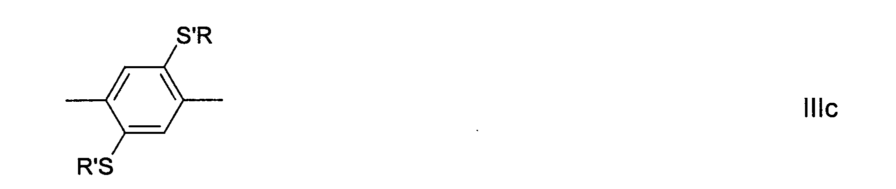 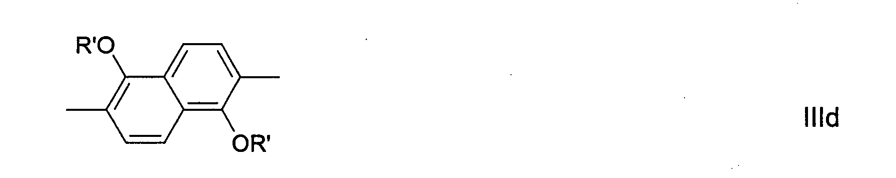 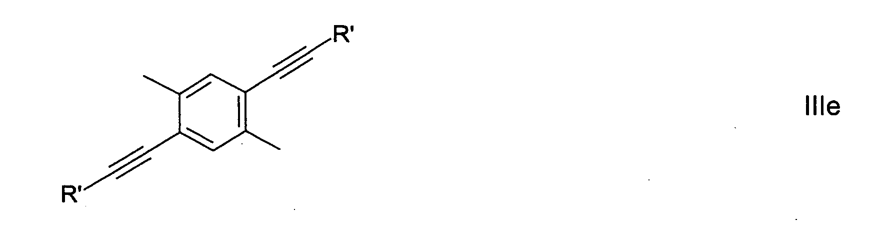 wherein R' has one of the meanings of R¹ in formula I.

12. Mono-, oligo- or polymer of formula la-c or I1a-c as defined in at least one of claims 2 to 11, with the provisos that
a) if X or Ar is unsubstituted thiophene-2,5-diyl, then at least one of R¹⁻⁴ is alkyl or cycloalkyl that is mono- or polysubstituted by F, Cl, Br, I or CN, optionally substituted aryl or heteroaryl, or P-Sp-, and
b) X and Ar(R¹R²) are different from dithienothiophene, 1,4-phenylene, 2,5-dialkyl- or 2,5-dialkoxy-1,4-phenylene, furan-2,5-diyl, 1-alkyl-1 H-pyrrol-2,5-diyl, 9H-fluorene-2,7-diyl, 9,9-dialkyl-9H-fluorene-2,7-diyl, N-alkyl-9H-carbazole-2,7-diyl and anthracene-9,10-diyl, and
c) Ar(R¹R²) is different from 2,5-dialkyl- or 2,5-dialkoxy-1,4-phenylene, naphthalene-2,6-diyl, naphthalene-4,8-diyl that is substituted in 1-, 4-, 5- and/or 8-position with alkoxy, dimethylsiloxane or oxymethyloxirane groups, 9,9-dialkyl-9H-fluorene-2,7-diyl and N-alkyl-9H-carbazole-2,7-diyl.

13. Polymerisable liquid crystal material comprising one or more mono-, oligo- or polymers as defined in at least one of claims 1 to 12 comprising at least one polymerisable group, and optionally comprising one or more further polymerisable compounds, wherein at least one of the polymerisable mono-, oligo- and polymers of claims 2 to 13 and/or the further polymerisable compounds is mesogenic or liquid crystalline.

14. Anisotropic polymer film with charge transport properties obtainable from a polymerisable liquid crystal material according to claim 15 that is aligned in its liquid crystal phase into macroscopically uniform orientation and polymerised or crosslinked to fix the oriented state.

15. Side chain liquid crystal polymer obtained by polymerisation of one or more mono- or oligomers or a polymerisable material as defined in at least one of claims 1 to 13 or by grafting one or more mono- or oligomers or a polymerisable material as defined in claims 1 to 13 to a polymer backbone in a polymeranaloguous reaction, optionally with one or more additional mesogenic or non-mesogenic comonomers.

16. Use of the mono-, oligo- and polymers, polymerisable materials and polymers as defined in at least one of claims 1 to 15 as semiconductors or charge transport materials in optical, electrooptical or electronic devices, like field effect transistors (FET) for example as components of integrated circuitry, of thin film transistors (TFT) for flat panel display applications, or of radio frequency identification (RFID) tags, or semiconducting components for organic light emitting diode (OLED) applications including both the charge transport and electroluminescent layers in electroluminescent displays or backlights of liquid crystal displays.

17. Use of the mono-, oligo- or polymer according to claim 12 as electroluminescent material, in photovoltaic or sensor devices, as electrode material in batteries, as photoconductor and for electrophotographic applications like electrophotographic recording.

18. Optical, electrooptical or electronic device, FET, integrated circuit (IC), TFT or OLED comprising a material, component or device according to at least one of claims 1 to 15.

19. TFT or TFT array for flat panel displays, radio frequency identification (RFID) tag, electroluminescent display or backlight, comprising a material, component or device according to at least one of claims 1 to 15 or a FET, IC, TFT or OLED according to claim 18.

20. Security marking or device comprising a FET or an RFID tag according to claim 19.

21. Mono-, oligo- and polymer, material or polymer as defined in at least one of claims 1 to 15, which is oxidatively or reductively doped to form conducting ionic species.

22. Charge injection layer, planarising layer, antistatic film or conducting substrate or pattern for electronic applications or flat panel displays, comprising a mono-, oligo- or polymer, material or polymer according to claim 21.
